# FASCICULE DE BREVET EUROPEEN

(11) **EP 2 069 821 B1**
(45) Date de publication et mention de la délivrance du brevet: **01.01.2020**
(21) Numéro de dépôt: 07823682.5
(22) Date de dépôt: 03.08.2007
(51) Int. Cl.: G01S 15/89, G01S 7/52, A61B 8/08, A61B 5/00, A61B 8/00

(54) **SONDE D'IMAGERIE ULTRASONORE POUR IMAGER UNE MODIFICATION TRANSITOIRE D'UN MILIEU**
ULTRASCHALL-BILDGEBUNGSSONDE ZUR ABBILDUNG EINER TEMPORÄREN VERÄNDERUNG IN EINER UMGEBUNG
ULTRASOUND IMAGING PROBE FOR IMAGING A TEMPORARY CHANGE IN AN ENVIRONMENT

(30) Priorité: 22.08.2006 FR 0607438; 03.01.2007 US 883243 P
(43) Date de publication de la demande: 17.06.2009
(73) Titulaire: SUPERSONIC IMAGINE, 13857 Aix-en-Provence Cedex 3 (FR)
(72) Inventeur: BERCOFF, Jérémy, 13090 Aix-en-Provence (FR); COHEN-BACRIE, Claude, 13090 Aix-en-Provence (FR); SOUQUET, Jacques, 13540 Puyricard (FR)
(74) Mandataire: Cabinet Beau de Loménie
(86) Numéro de dépôt international: PCT/FR2007/051773
(87) Numéro de publication internationale: WO 2008/023127

(56) Documents cités:
- FR-A1- 2 791 136
- FR-A1- 2 844 058
- FR-A1- 2 844 178
- US-A- 5 810 731
- US-A- 6 068 597
- US-A1- 2004 068 184
- US-B2- 6 984 209

## Description

### Arrière-plan de l'invention

La présente invention se rapporte au domaine général des sondes destinées à l'imagerie ultrasonore, également nommée « imagerie échographique ».

L'invention est plus particulièrement relative aux procédés et sondes permettant l'imagerie des propriétés viscoélastiques basées sur l'utilisation d'une pression de radiation ultrasonore.

Les sondes échographiques classiques sont conçues pour, à la fois, transmettre des ondes ultrasonores dans les tissus dans un milieu et capter des signaux rétrodiffusés afin de les analyser et reformer une image du milieu.

Typiquement, ces sondes sont composées d'une série de N transducteurs piézoélectriques alignés le long d'une ligne. Cette ligne peut être droite ou courbée.

Les transducteurs piézoélectriques sont pilotés individuellement par des voies électroniques capables d'appliquer des signaux électriques déphasés les uns par rapport aux autres.

En ajustant les phases et/ou retards selon une loi cylindrique, il est possible de focaliser un faisceau ultrasonore à un endroit donné dans le milieu, en créant ainsi électroniquement l'équivalent d'une lentille. Ces lois sont aussi utilisées aux étapes de réception pour isoler les signaux rétrodiffusés provenant d'un endroit donné du milieu et reconstituer son image acoustique.

La taille et l'espacement des transducteurs dépend de la fréquence de la sonde ultrasonore et varie typiquement entre 0,5 et 1 longueur d'onde de l'onde ultrasonore émise.

Avec une telle sonde unidimensionnelle, la focalisation électronique et la reconstruction de l'image ultrasonore ne peut se réaliser que dans un plan.

Dans la troisième dimension, appelée « élévation », il est d'usage d'appliquer sur les transducteurs piézoélectriques une lentille géométrique fixe permettant de confiner le faisceau ultrasonore sur une tranche d'épaisseur raisonnable.

Ainsi, typiquement, la taille des transducteurs piézoélectriques en élévation est de 20 longueurs d'ondes de l'onde ultrasonore émise et la profondeur de focalisation géométrique de 100 longueurs d'ondes de l'onde ultrasonore émise.

Les ultrasons sont parfois utilisés pour créer des modifications transitoires dans le milieu, par exemple une pression de radiation ultrasonore.

L'utilisation de la pression de radiation ultrasonore est utilisée dans les techniques élastographiques. Ces techniques sont des modes d'imagerie supplémentaire à celui d'une imagerie échographique classique.

Cependant, l'utilisation de barrettes échographiques standards conçues particulièrement pour fournir une image échographique de très haute qualité ne sont pas optimales pour la mise en œuvre des techniques élastographiques et plus généralement, la réalisation de modifications transitoires au sein du milieu.

Les propriétés géométriques et acoustiques des sondes connues ne sont pas adaptées pour la génération de contraintes mécaniques internes.

En outre, la qualité des images élastographiques résultantes n'est pas satisfaisante.

Dans le cas des techniques élastographiques, les limitations induites par les sondes connues sont au nombre de trois.

Tout d'abord, la profondeur de pénétration de la contrainte mécanique est limitée, généralement à la moitié de la profondeur potentiellement interrogeable.

Ensuite, la largeur de la zone d'exploration est également limitée parce que la source de vibration mécanique interne présente une géométrie inadaptée.

Enfin, des champs acoustiques très intenses sont créés pour permettre la génération de la contrainte mécanique interne.

L'intensité de ces champs acoustiques peut dépasser les limites d'exposition en vigueur et être dangereux pour les patients.

US 2004/068184 (A1) divulgue un appareil d'imagerie ave une source de cisaillement virtuellement agrandi.

US 6,068,597 divulgue une sonde comprenant un transducteur ultrasonore entouré par deux transducteurs auditifs.

US 6,984,209 divulgue une sonde comprenant un transducteur de diagnostique entouré par deux transducteurs de transmission.

### Objet et résumé de l'invention

La présente invention a donc pour but principal de palier à tels inconvénients en proposant une solution pour générer une contrainte mécanique interne optimale tout en satisfaisant les puissances acoustiques réglementaires et en ne faisant aucun compromis sur la qualité de l'imagerie échographique.

L'invention concerne une sonde d'imagerie ultrasonore pour imager un milieu selon la revendication 1.

La sonde comprend deux types de transducteur(s) fonctionnant à des fréquences distinctes, caractérisé en ce que le premier type de transducteur(s) est dédié à l'imagerie ultrasonore du milieu, et le second type de transducteur(s) est dédié à la génération d'une contrainte engendrant au moins une modification transitoire du milieu imagé, les deux types de transducteur(s) étant aptes à fonctionner au moins dans un mode dit couplé où le premier type de transducteurs fonctionne de manière synchronisée avec le second type de transducteurs de façon à imager l'évolution de la modification transitoire du milieu.

Avec une telle sonde, le second type de transducteur est adapté à la génération d'une modification transitoire du milieu et synchronisé avec le premier type de transducteur destiné à imager cette modification transitoire. La synchronisation des deux types de transducteurs est réalisée en fonction des propriétés physiques et cinétiques de l'évolution de la modification transitoire du milieu. Les dispositions relatives des transducteurs peuvent aussi être fonction de ces propriétés.

Selon un mode de réalisation de l'invention, les deux types de transducteurs sont distincts par leurs caractéristiques géométriques et acoustiques.

Selon l'invention les deux types de transducteurs fonctionnent à des fréquences distinctes.

Les premiers transducteurs étant dédiés à l'imagerie ultrasonore, cela permet d'obtenir des images échographiques de grande qualité.

Ces images échographiques sont avantageusement des images échographiques classiques et des images échographiques des mouvements transitoires, en particulier les images d'un mouvement en cisaillement permettant de faire une mesure élastographique.

Ainsi, avantageusement le premier type de transducteurs a deux modes de fonctionnement, le mode dit couplé et un mode dit classique où le premier type de transducteurs réalise une image échographique du milieu.

Selon une caractéristique particulière de l'invention, la contrainte engendrant une modification transitoire est propagative, le second type de transducteur étant alors synchronisé en tenant compte des caractéristiques de la propagation de la contrainte engendrant la modification transitoire.

Avec une telle caractéristique, il est possible de visualiser directement et simplement la propagation d'une onde dans le milieu.

Avantageusement, la contrainte engendrant une modification transitoire est une contrainte mécanique par pression de radiation ultrasonore.

Une telle contrainte permet de réaliser des mesures d'élastographie permettant de caractériser les propriétés élastiques du milieu.

Dans une réalisation de l'invention, les transducteurs dédiés à l'imagerie ultrasonore sont positionnés de manière linéaire. Cette réalisation correspond à un des formats habituels des sondes d'imagerie et l'implémentation de l'invention dans une sonde similaire à l'existant autorise une prise en main rapide des praticiens. La ligne définie par l'alignement des transducteurs peut être droite ou courbée ou encore prendre une forme adaptée aux caractéristiques géométriques du milieu à observer.

Selon l'invention les transducteurs dédiés à la génération de la contrainte engendrant une modification transitoire sont répartis en deux lignes disposées de part et d'autre des transducteurs dédiés à l'imagerie.

De nouveau, la disposition de ces derniers transducteurs pourra être selon un alignement droit ou courbé ou encore ils pourront être placés selon une forme adaptée aux caractéristiques géométriques du milieu à observer.

Selon l'invention, les transducteurs dédiés à la génération de la contrainte engendrant une modification transitoire du milieu ont une focalisation géométrique en élévation plus lointaine que les transducteurs dédiés à l'imagerie.

Avec une telle caractéristique, le volume de la zone de contrainte est augmenté, la qualité de la contrainte est améliorée et l'énergie locale déposée dans le milieu est diminuée.

Selon l'invention, les transducteurs dédiés à la génération de la contrainte engendrant une modification transitoire du milieu ont une fréquence de résonance plus faible que celle des transducteurs dédiés à l'imagerie.

Dans le cas où on génère une pression de radiation, cette dernière sera d'autant plus efficace et d'autant plus profonde grâce à cette caractéristique.

Dans un mode de réalisation dans lequel les transducteurs sont munis de lentilles de focalisation en élévation, ces lentilles sont indépendantes pour les deux types de transducteurs. Ces lentilles de focalisation peuvent être implémentées sous la forme d'une seule lentille présentant deux courbures différentes.

Dans une mise en oeuvre de l'invention, les transducteurs des deux types sont contrôlés par des voies électroniques indépendantes et aptes à être contrôlées de manière synchrone.

### Brève description des dessins

D'autres caractéristiques et avantages de la présente invention ressortiront de la description faite ci-dessous, en référence aux dessins annexés qui en illustrent un exemple de réalisation dépourvu de tout caractère limitatif. Sur les figures :
- les figures 1a et 1b représentent schématiquement une sonde selon l'invention,
- les figures 2a et 2b montrent l'allure des champs de pression respectivement obtenus avec une sonde classique et une sonde selon l'invention,
- la figure 3 représente l'amplitude des champs de pression en fonction de la profondeur obtenue avec une sonde classique et une sonde selon l'invention,
- la figure 4 montre l'atténuation du champ de cisaillement créé par les champs de pression de la figure 3,
- les figures 5a à 5c illustrent l'effet de focalisation pour trois focalisations différentes obtenu avec une sonde classique et une sonde selon l'invention.

### Description détaillée d'un mode de réalisation

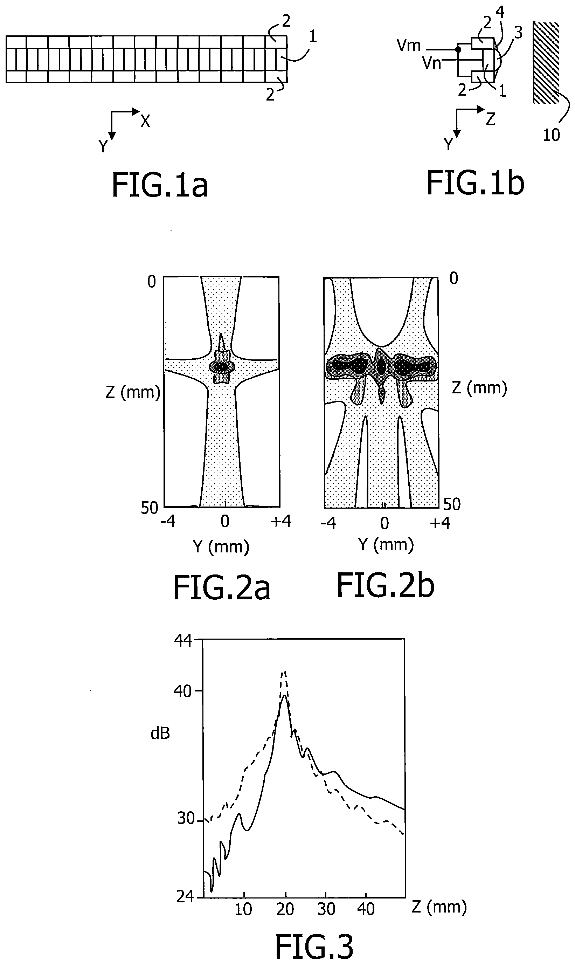

La figure 1 décrit un exemple de réalisation d'une sonde selon l'invention. La sonde décrite est destinée à une application couplant l'imagerie échographique et l'imagerie élastographique. Plus particulièrement, la sonde pourrait être utilisée pour l'imagerie du sein.

La sonde décrite s'étend le long d'une dimension X et présente deux types de transducteurs 1 et 2.

Un premier type de transducteurs 1, disposés de manière centrale sur la sonde et représentés en surface hachurée, est destiné à l'imagerie. Les transducteurs 1 sont, par exemple, au nombre de 256. Ils présentent avantageusement une fréquence de résonance de 8 MHz et une largeur de 0,2 mm, sur la dimension X, sur une hauteur de 4 mm, sur la dimension Y. On note que, par clarté, sur la figure 1, les échelles en X et en Y sont différentes. Ils sont pilotés par 128 voies électroniques indépendantes, via un multiplexeur inséré dans la sonde elle-même ou placés dans un système échographique auquel est reliée la sonde. Avec de telles caractéristiques, les transducteurs 1 permettent une image échographique bidimensionnelle du sein de haute qualité.

Un second type de transducteurs 2 est destiné à la génération d'une contrainte mécanique interne permettant la propagation d'une onde de cisaillement dans le milieu. Ils sont disposés linéairement de part et d'autre des transducteurs 1. Ces transducteurs 2 sont au nombre de 256, soit 128 transducteurs de chaque côté de la ligne constituée par les transducteurs 1. Ces transducteurs 2 ont une fréquence de résonance égale à la moitié de celle des transducteurs de type 1, c'est-à-dire une fréquence de résonance égale à 4 MHz. Ils présentent une largeur double de 0,4 mm et une hauteur de moitié, c'est-à-dire 2 mm.

Les deux types de transducteurs appartiennent à la famille des transducteurs ultrasonores. Leurs fréquences de résonance sont supérieures à 20 kHz mais appartiennent à des intervalles de fréquences distincts. Les deux types de transducteurs peuvent ainsi se différencier par des fréquences ultrasonore de résonance distinctes et par des propriétés géométriques distinctes, notamment leurs tailles respectives.

Bien que, sur l'exemple de la figure 1, le nombre de transducteurs dédiés à la génération de la contrainte est le même que le nombre de transducteurs d'imagerie, on fait ici remarquer que cette caractéristique n'est pas limitative, ces nombres pouvant être différents.

Comme illustré sur la coupe de la sonde représentée sur la figure 1b, chaque paire de transducteurs 2 situés de part et d'autre des transducteurs 1 sont couplés électroniquement et pilotés par la même voie électronique. Les 256 transducteurs de type 2 sont donc pilotés par 128 voies électroniques différentes de celles qui pilotent les transducteurs de type 1.

La sonde obtenue est donc pilotée par un système échographique possédant 256 voies électroniques indépendantes.

Au-dessus des transducteurs 1 et 2 sont respectivement placées des lentilles 3 et 4 qui permettent la focalisation en élévation, respectivement des champs de poussée et des champs d'imagerie. On souligne ici que les lentilles 3 et 4 peuvent aussi être des parties d'une seule et même lentille présentant deux courbures différentes. La focalisation définie par la courbure de la lentille est différente pour les transducteurs 1 et les transducteurs 2. Pour les transducteurs 1, la focalisation est réalisée à 20 mm alors que pour les transducteurs 2, la focalisation est réalisée à 60 mm.

L'utilisation de focalisations différentes permet d'étaler dans l'espace le champ ultrasonore destiné à la génération de la contrainte tout en gardant un champ confiné optimal pour l'imagerie échographique.

Les deux types de transducteurs sont commandés de manière synchrone de façon à imager, avec les transducteurs 1, l'évolution de la modification transitoire du milieu provoquée par les transducteurs 2.

Avantageusement, les transducteurs 1 ont deux modes de fonctionnement, un premier mode dit classique où le premier type de transducteurs réalise une simple image échographique du milieu, un second mode dit couplé où le premier type de transducteurs fonctionne de manière synchronisée avec le second type de transducteurs de manière à imager l'évolution de la modification transitoire du milieu.

La synchronisation des deux types de transducteurs est avantageusement réalisée selon les principes décrits dans la demande de brevet française publiée sous le numéro FR 2 844 058.

Dans la mesure où les transducteurs 1 présentent les mêmes caractéristiques géométriques et acoustiques que celles d'une sonde linéaire échographique standard, les performances d'une telle sonde sont identiques, du point de vue échographique, à celles d'une sonde linéaire ayant les caractéristiques des transducteurs centraux décrits auparavant.

On constate que ces caractéristiques correspondent à celles utilisées dans le cadre de l'échographie mammaire.

Dans la suite, sont analysées les performances d'une telle sonde pour l'élastographie.

La figure 2a montre un champ de pression dans le plan (Y,Z) obtenu avec une sonde classique unidimensionnelle présentant une focalisation à 20 mm.

La figure 2b montre le champ de pression dans le même plan (Y,Z) obtenu avec une sonde selon l'invention telle que représentée sur la figure 1.

Sur ces figures, l'intensité du champ de pression est représentée d'une manière d'autant plus foncée qu'elle est importante. On constate que, sur la figure 2b, le champ de pression est beaucoup plus étalé sur la direction Y que dans le cas d'une sonde unidimensionnelle classique représentée sur la figure 2a.

Ainsi, la contrainte générée par la sonde selon l'invention se révèle être à la fois plus intense, car le maximum de pression est observé sur une zone plus étendue, et mieux répartie. Cela correspond à la satisfaction des objectifs poursuivis, à savoir une intensification du champ de pression et, par voie de conséquence, à la génération d'une onde de cisaillement particulièrement adaptée à l'élastographie.

La figure 3 représente l'amplitude des champs de pression obtenus en fonction de la profondeur, au point de coordonnées (0,0,Z), avec Z variant de 0 à 50 mm.

En pointillés, est représentée l'amplitude en décibels du champ de pression obtenu par la sonde classique unidimensionnelle et, en traits pleins, est représentée l'amplitude du champ de pression pour la sonde selon l'invention.

On remarque qu'au point de focalisation à 20 mm, le champ de pression est plus faible de 3 décibels pour la sonde selon l'invention par rapport à la sonde unidimensionnelle classique.

Néanmoins, ce paramètre est moins important pour la mise en œuvre d'un procédé élastographique que la longueur de propagation de l'onde de cisaillement créé par le champ de pression.

Les champs de pression évoqués ci-dessus créent en effet une source de cisaillement.

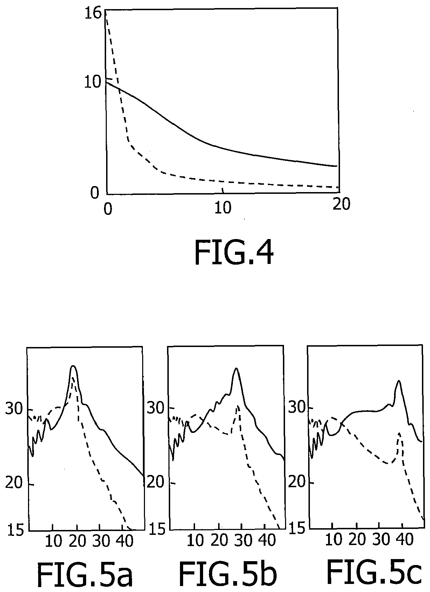

Sur la figure 4, l'atténuation du champ de cisaillement créé par les champs de pression évoqués ci-dessus est représentée comme un déplacement D en fonction de la distance latérale X à la source. Cela permet de comparer les ondes de cisaillement résultantes pour des deux sondes avec une focalisation à 20 mm.

On note que si le champ de déplacement est moins important au centre de la source avec la sonde selon l'invention (trait plein), il s'atténue beaucoup moins vite que pour la sonde classique (trait pointillé) et est même quatre fois plus intense après deux centimètres de propagation.

Ceci est dû à l'étalement de la source de cisaillement dans la direction élévationnelle Y ainsi que représenté sur la figure 2b. Cela permet au champ de cisaillement d'être moins diffractant en dehors du plan de l'imagerie.

Ainsi, l'invention permet de générer une onde de cisaillement de meilleure qualité tout en induisant localement un champ de pression moins intense. Dans les cas où les limites règlementaires des puissances acoustiques sont contraignantes, cela peut se révéler être fort avantageux.

En outre, il est nécessaire de s'intéresser à la profondeur de pénétration de la source de cisaillement pour mettre en œuvre, de manière satisfaisante, un procédé élastographique. En effet, pour utiliser un procédé élastographique de manière satisfaisante, il est nécessaire de focaliser et de créer une onde de cisaillement aussi profonde que la profondeur par ailleurs imagée par échographie.

Cela impose que les transducteurs 2 soient des transducteurs fonctionnant à une fréquence plus basse que les transducteurs 1 destinés à l'échographie. Sinon, comme c'est le cas avec une sonde classique, l'effet de focalisation est limité à environ la moitié de la profondeur d'imagerie et ce, à cause de l'atténuation ultrasonore.

Les figures 5a à 5c illustrent cela pour des focalisations respectivement à 20, 30 et 40 mm de chacune des sondes, classiques et selon l'invention. Ces figures représentent les champs de pressions créés par les deux sondes sur une profondeur en Z de 50 mm.

On constate que la sonde selon l'invention (trait plein) permet une poussée dans le milieu à plus de 40 mm, alors que la profondeur de pénétration de la sonde classique est de l'ordre d'une vingtaine de millimètres.

On remarque enfin que diverses mises en œuvre peuvent être réalisées par l'homme du métier selon les principes de l'invention définis dans les revendications qui suivent. Notamment la disposition des transducteurs peut être variée. Les transducteurs 1 et 2 peuvent ainsi être superposés l'un sur l'autre. Dans ce cas, seuls les transducteurs 1 d'imagerie restent visibles pour l'opérateur, les transducteurs 2 étant placés « derrière » les transducteurs 1 et par conséquent masqués par ceux-ci. Le nombre et la forme des transducteurs de chaque sorte peuvent également être divers. Il est possible d'utiliser plus de transducteurs 1 et moins de transducteurs 2 et inversement.

On constate aussi que, bien que spécifiquement adapté à la génération de contrainte, les transducteurs du second type, ou une partie d'entre eux, peuvent aussi être utilisés pour réaliser de l'imagerie échographique en mode couplé, par exemple avant et après la génération de la contrainte, et ce de manière à couvrir une zone d'imagerie plus large dans la direction élévationnelle qu'avec les seuls transducteurs du premier type. Avec l'exemple de sonde proposé sur la figure 1, il devient alors possible d'imager simultanément en mode couplé dans trois plans d'imagerie distincts.

On constate aussi que les transducteurs du premier type, ou une partie d'entre eux, peuvent aussi être utilisés pour générer une contrainte en complément de celle spécifiquement générée par les transducteurs du second type.

## Revendications

1. Sonde d'imagerie ultrasonore pour imager un milieu (10), la sonde comprenant :
- une pluralité de transducteurs (1) d'un premier type configurés pour l'imagerie ultrasonore du milieu (10) et ayant une première focalisation dans le milieu, et
- une pluralité de transducteurs ultrasonores (2) d'un second type configurés pour la génération d'une contrainte engendrant au moins une modification transitoire du milieu (10) et ayant une seconde focalisation dans le milieu, les transducteurs des deux types (1, 2) étant configurés pour fonctionner au moins dans un mode couplé où le premier type de transducteurs (1) fonctionne de manière synchronisée avec le second type de transducteurs (2) de façon à imager l'évolution de la modification transitoire du milieu (10),
ladite sonde étant **caractérisée en ce que** :
les transducteurs (2) dédiés à la génération de la contrainte engendrant une modification transitoire sont répartis en deux lignes disposées de part et d'autre des transducteurs (1) dédiés à l'imagerie,
les transducteurs du premier et second type (1, 2) ont des focalisations différentes configurées de telle manière que les transducteurs (2) dédiés à la génération de la contrainte engendrant une modification transitoire du milieu ont une focalisation géométrique en élévation plus lointaine que les transducteurs (1) dédiés à l'imagerie,
les transducteurs (2) dédiés à la génération de la contrainte engendrant une modification transitoire du milieu ont une fréquence de résonance plus faible que celle des transducteurs (1) dédiés à l'imagerie.

2. Sonde selon la revendication 1, **caractérisée en ce que** les deux types de transducteurs (1, 2) sont distincts par leurs caractéristiques géométriques et acoustiques.

3. Sonde selon l'une des revendications 1 et 2, **caractérisée en ce que** la contrainte engendrant une modification transitoire est propagative, le second type de transducteur (2) étant alors synchronisé en tenant compte des caractéristiques de la propagation de la contrainte engendrant la modification transitoire.

4. Sonde selon la revendication 3, **caractérisée en ce que** la contrainte engendrant une modification transitoire est une contrainte mécanique par pression de radiation ultrasonore.

5. Sonde selon l'une des revendications précédentes, **caractérisée en ce que** les transducteurs (1) dédiés à l'imagerie ultrasonore sont positionnés de manière linéaire.

6. Sonde selon l'une des revendications précédentes, **caractérisée en ce que**, les transducteurs (1, 2) sont munis de lentilles de focalisation en élévation, ces lentilles étant indépendantes pour les deux types de transducteurs (1, 2).

7. Sonde selon l'une des revendications précédentes, **caractérisée en ce que** les transducteurs (1, 2) des deux types sont contrôlés par des voies électroniques (Vn, Vm) indépendantes et apte à être contrôlées de manière synchrone.

## Patentansprüche

1. Ultraschallbildgebungssonde zum Abbilden einer Umgebung (10), wobei die Sonde aufweist:
- mehrere Wandler (1) eines ersten Typs, die für die Ultraschallbildgebung der Umgebung (10) konfiguriert sind und eine erste Fokussierung in der Umgebung aufweisen, und
- mehrere Ultraschallwandler (2) eines zweiten Typs, die für das Erzeugen einer Beanspruchung konfiguriert sind, die mindestens eine transitorische Änderung der Umgebung (10) hervorruft, und eine zweite Fokussierung in der Umgebung aufweisen, wobei die Wandler der zwei Typen (1, 2) konfiguriert sind, um mindestens in einem verbundenen Modus zu funktionieren, in dem der erste Typ von Wandlern (1) synchronisiert mit dem zweiten Typ von Wandlern (2) derart funktioniert, um die Entwicklung der vorübergehenden Änderung der Umgebung (10) abzubilden,
wobei die Sonde **dadurch gekennzeichnet ist, dass**:
die Wandler (2), die dem Erzeugen der Beanspruchung, die eine vorübergehende Änderung hervorruft, gewidmet sind, auf zwei Linien verteilt sind, die auf beiden Seiten der Wandler (1) angeordnet sind, die der Bildgebung gewidmet sind, die Wandler des ersten und zweiten Typs (1, 2) verschiedene Fokussierungen aufweisen, die derart konfiguriert sind, dass die Wandler (2), die dem Erzeugen der Beanspruchung gewidmet sind, die eine transitorische Änderung der Umgebung hervorruft, eine weiter entfernte geometrische Fokussierung in Höhenrichtung als die Wandler (1) aufweisen, die der Bildgebung gewidmet sind,
die Wandler (2), die dem Erzeugen der Beanspruchung gewidmet sind, die eine vorübergehende Änderung der Umgebung hervorruft, eine niedrigere Resonanzfrequenz als jene der Wandler (1) aufweisen, die der Bildgebung gewidmet sind.

2. Sonde nach Anspruch 1, **dadurch gekennzeichnet, dass** sich die zwei Typen von Wandlern (1, 2) durch ihre geometrischen und akustischen Merkmale unterscheiden.

3. Sonde nach einem der Ansprüche 1 und 2, **dadurch gekennzeichnet, dass** die Beanspruchung, die eine transitorische Änderung hervorruft, propagativ ist, wobei der zweite Typ von Wandler (2) dann synchronisiert wird, indem die Ausbreitungseigenschaften der Beanspruchung, die die vorübergehende Änderung hervorruft, berücksichtigt werden.

4. Sonde nach Anspruch 3, **dadurch gekennzeichnet, dass** die Beanspruchung, die eine vorübergehende Änderung hervorruft, eine mechanische Beanspruchung durch Ultraschallstrahlungsdruck ist.

5. Sonde nach einem der vorhergehenden Ansprüche, **dadurch gekennzeichnet, dass** die Wandler (1), die der Ultraschallbildgebung gewidmet sind, linear angeordnet sind.

6. Sonde nach einem der vorhergehenden Ansprüche, **dadurch gekennzeichnet, dass** die Wandler (1, 2) mit Fokussierlinsen in Höhenrichtung ausgestattet sind, wobei diese Linsen für die zwei Typen von Wandlern (1, 2) unabhängig sind.

7. Sonde nach einem der vorhergehenden Ansprüche, **dadurch gekennzeichnet, dass** die Wandler (1, 2) der zwei Typen durch unabhängige elektronische Mittel (Vn, Vm) gesteuert werden und geeignet sind, synchron gesteuert zu werden.

## Claims

1. An ultrasonic imaging probe for imaging a medium (10), the probe comprising:
- a plurality of transducers (1) of a first type dedicated to ultrasonic imaging of the medium (10) and having a first focus in the medium, and
- a plurality of ultrasonic transducers (2) of a second type dedicated to generating a stress producing at least one transient modification of the medium (10) and having a second focus in the medium, the transducers of both types (1, 2) being able to operate at least in a coupled mode where the first type of transducers (1) operates in a synchronized way with the second type of transducers (2) so as to image the time course of the transient modification of the medium (10),
said probe being **characterized in that**:
the transducers (2) dedicated to generating the stress producing a transient modification are then distributed in two lines positioned on either side of the transducers (1) dedicated to imaging,
the transducers of the first and second type (1, 2) have different focuses configured so that the transducers (2) dedicated to generating the stress producing a transient modification of the medium have a more remote elevational geometrical focus than the transducers (1) dedicated to imaging,
the transducers (2) dedicated to generating the stress producing a transient modification of the medium have a lower resonance frequency than that of the transducers (1) dedicated to imaging.

2. The probe according to claim 1, **characterized in that** both types of transducers (1, 2) are distinct by their geometrical and acoustic characteristics.

3. The probe according to any of claims 1 and 2, **characterized in that** the stress generating a transient modification is propagative, the second of transducers (2) being then synchronized while taking into account the characteristics of the propagation of the stress producing the transient modification.

4. The probe according to claim 3, **characterized in that** the stress producing a transient modification is a mechanical stress by ultrasonic radiation pressure.

5. The probe according to any of the preceding claims, **characterized in that** the transducers (1) dedicated to ultrasonic imaging are positioned linearly.

6. The probe according to any of the preceding claims, **characterized in that** the transducers (1, 2) are provided with elevational focussing lenses, these lenses being independent for both types of transducers (1, 2).

7. The probe according to any of the preceding claims, **characterized in that** the transducers (1, 2) of both types are controlled via independent electronic routes (Vn, Vm) and may be controlled synchronously.
